# EUROPEAN PATENT APPLICATION

(11) **EP 2 641 560 A1**
(43) Date of publication of application: **25.09.2013**
(21) Application number: 11840824.4
(22) Date of filing: 02.11.2011
(51) Int. Cl.: A61B 19/00

(54) **SURGICAL PAD**

(30) Priority: 18.11.2010 JP 2010257427
(71) Applicant: Nakamura, Shoichi, Higashichikuma-gun, Nagano 399-7502 (JP); ACP Japan Co. Ltd., Tokyo 113-0033 (JP)
(72) Inventor: NAKAMURA, Shoichi, Nagano 399-7502 (JP)
(74) Representative: Gassner, Wolfgang
(86) International application number: PCT/JP2011/075269
(87) International publication number: WO 2012/066932

(57) **Abstract**

To provide a surgical pad which does not cause waste threads and fluff on the pad surface to remain in an operation site during an operation, is not hardened in absorbing body fluids such as lymph, blood and cerebrospinal fluid, and which is detectable by X-ray contrast after the operation, the surgical pad, which absorbs body fluids such as lymph, blood and cerebrospinal fluid during surgery, is provided with a pad portion formed by layering a plurality of pieces of cloth having absorption, and a radiopaque substance containing sheet disposed between layered pieces of cloth or on the surface of the pad portion. Herein, by coating the surface of the pad portion with resin, waste threads and fluff of the pad surface are prevented from peeling off.

## Description

### Technical Field

The present invention relates to a surgical pad to absorb body fluids such as blood, lymph and spinal fluid occurring from a site under an operation in surgery, and more particularly, to a surgical pad to effectively absorb a cerebrospinal fluid and the like oozing from the ventricles of the brain and subarachnoid space inside the skull without injuring brain cells in brain surgery.

### Background Art

In the medical field of surgery, absorption supplies such as cut cotton, gauze and surgical pad are used when necessary to absorb and remove body fluids such as blood, lymph and cerebrospinal fluid.

In the case of performing brain surgery and orthopedic surgery, absorption supplies of relatively small sizes are used to absorb body fluids such as lymph, blood and cerebrospinal fluid. However, the absorption supply of the small size is difficult to distinguish from tissue of the operation site after absorbing body fluids or the like, and is sometimes left inside the body erroneously after the operation.

In this case, for example, in the case of brain surgery, even when the pad left inside the skull is of any small size, since there is a risk that the pad presses minute brain cells to cause various kinds of damage, and that immune antibody action of the human body causes inflammation in the intracerebral side to eliminate the pad, and various physical discomforts such as pain, uncomfortable feeling and slight fever are derived, such an absorption material should be removed after finishing the operation, and in case that the pad is left inside the body, it is necessary to detect the pad.

Therefore, particularly, for the surgical pad, such a measure has conventionally been taken that a thread is sewn so that the thread is a mark of a different color to indicate clearly that the surgical pad exists in the operation site during the operation and is to pull out in removing the pad from the body and that the other end of the thread is extended long outside the body (for example, see Patent Documents 1 and 2).

Further, in Patent Document 1, to ensure removal of a surgical pad, such a pad is known that a contrast thread (radiopaque resin thread) is attached to a pad portion, and that X-ray contrast (X-ray photography) is performed before closing the operation site in the final phase of the operation to check the presence or absence of existence of the surgical pad using the contrast thread as a cue.

### Prior Art Document

### Patent Document

Patent Document 1: Japanese Patent Application Publication No. 2002-325774
Patent Document 2: Japanese Patent Application Publication No. 2009-061186

### Summary of Invention

### Problems to be Solved by the Invention

As such a contrast thread for X-ray contrast after an operation, a thread-shaped filament has conventionally been used. The thread-shaped filament is obtained by twisting a thread-shaped material comprised of a filament made of barium sulfate having contrast action and polypropylene resin.

However, such a thread-shaped filament tends to absorb liquid due to the structure, and the material of the contrast thread is resin, and has the property of absorbing moisture to be hardened. Therefore, when the hardened contrast thread contacts tissue in the body, there is a risk of injuring the tissue, particularly, delicate brain tissue in brain surgery.

Then, when the contrast thread is thinned, too thin a thread is hard to find in X-ray contrast, and the thickness to some extent is required. However, when such a contrast thread absorbs moisture and is hardened, the large lump-shaped substance appears in the pad, and there is a risk of injuring the surface of tissue in the body.

Further, also for the pad portion, since a fine thread material tends to drop from the cut end portion when the pad is gauze or absorbent cotton, as disclosed in Patent Document 1, the pad is formed by layering many pieces of nonwoven fabric. However, in even the pad using nonwoven fabric, it is not possible to completely prevent waste threads and thread fluff material from the surface from peeling off.

Therefore, it is an object of the present invention to provide a surgical pad which does not cause waste threads and fluff on the pad surface to remain in an operation site during an operation, is not hardened in absorbing body fluids such as lymph, blood and cerebrospinal fluid, and which is detectable by X-ray contrast after the operation.

### Means for Solving the Problem

To attain the aforementioned object, the present invention provides a surgical pad that absorbs body fluids such as lymph, blood and cerebrospinal fluid during surgery characterized by having a pad portion formed by layering a plurality of pieces of cloth having absorption, a coating portion formed from a resin on a surface of the pad portion, and a radiopaque substance containing sheet disposed between layered pieces of cloth or on the surface (in addition, in the invention, the "surface" means either of surfaces on both sides of the pad portion) of the pad portion. A radiopaque substance containing body is formed in the shape of a sheet, arranged in between layers of the cloth or on the surface of the pad, and therefore, is not hardened by absorbing blood, body fluid and the like.

The radiopaque substance containing sheet is characterized by being a film-shaped sheet with a thickness of the order of micrometers with the radiopaque substance kneaded in a base material. Meanwhile, the radiopaque substance containing sheet is characterized in that a coating of the radiopaque substance is applied to the surface of the base material, and that the base material used in the opaque substance containing sheet is made of a resin.

Further, the radiopaque substance containing sheet is characterized by being disposed between layered pieces of cloth or on the surface of the pad portion, and formed by containing the radiopaque substance in a sheet-shaped thermoplastic resin plate, and in that the thermoplastic resin plate is heat-sealed to the pad portion. By this means, the cloth and the radiopaque substance containing sheet is integrally held.

Furthermore, when the diameter of a particle of the resin material of the coating is 5 µmm or less, smoothness is improved. Then, when the particle is formed from a hydrophilic resin or a material containing a hydrophilic resin, it is possible to pull out the pad more easily.

It is a feature that the surface of the pad portion is coated with a resin. By this means, since the surface of the pad portion is covered with the coating of the resin material, it is possible to prevent waste threads and fluff on the surface from peeling off. Accordingly, the cloth may be nonwoven fabrics or woven fabrics, and it is possible to select fabrics excellent in functions of absorption and the like. Then, the resin material of the coating is characterized by containing an antibacterial agent.

Further, it is a feature that a drawing thread for a mark is attached to the pad portion. When a natural material, particularly, silk is used for the drawing thread, the drawing thread is softened in absorbing blood and/or body fluid, and friction with tissue in the body is low.

### Advantageous Effect of the Invention

According to the surgical pad in the invention, the radiopaque substance containing body is made a sheet, and is thereby not hardened by absorbing body fluids such as lymph, blood and cerebrospinal fluid occurring in an operation, and further, the pad does not have any asperities, thereby has low friction with tissue in the body, and is prevented from injuring the tissue. Furthermore, in X-ray contrast, since the radiopaque substance is captured on the surface of the sheet, it is possible to detect the surgical pad reliably.

### Brief Description of Drawings

FIG. 1 is a diagram to explain a configuration of a surgical pad according to one Embodiment of the invention;
FIG. 2 contains diagrams showing a part of A-A cross section of the surgical pad shown in FIG. 1;
FIG. 3 contains diagrams to explain an example of a configuration in cross section that a radiopaque substance containing sheet contains a radiopaque substance;
FIG. 4 contains diagrams to explain a configuration of a surgical pad according to another Embodiment of the invention;
FIG. 5 contains diagrams to explain various modifications of the shape of the pad and an arrangement relationship between a drawing thread and the radiopaque substance containing sheet in the surgical pad of the invention;
FIG. 6 contains diagrams to explain other modifications of the shape of the pad and the arrangement relationship between the drawing thread and the radiopaque substance containing sheet in the surgical pad of the invention; and
FIG. 7 contains diagrams to explain modifications of the shape of cross section of the radiopaque substance containing sheet. Mode for Carrying Out the Invention

One Embodiment for embodying the invention will be described based on drawings.

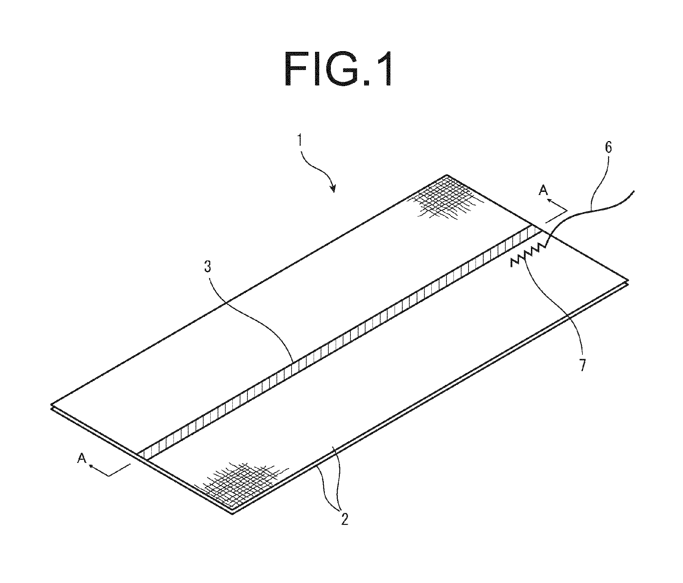

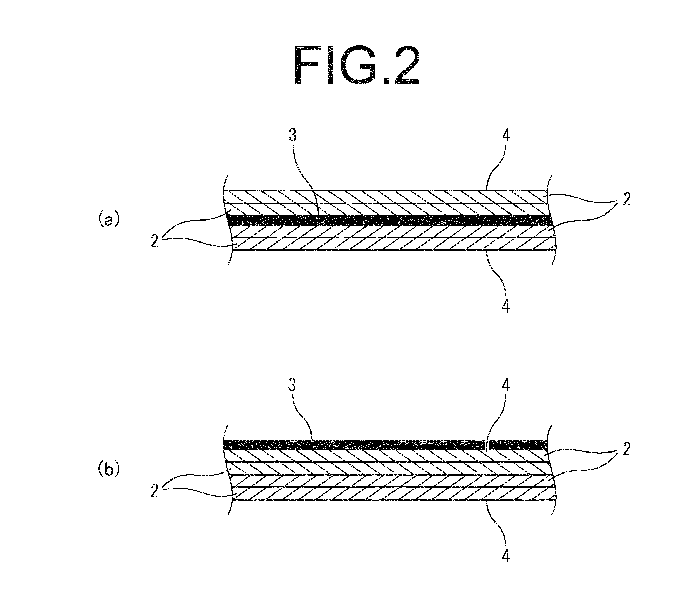

FIG. 1 shows a configuration of a surgical pad according to the Embodiment, and FIG. 2 shows a part of cross section taken along by A-A of FIG. 1. A pad portion 1 is formed by layering and integrating a plurality of pieces of cloth 2 (for example, 60 mm x 30 mm) having absorption, and a coating 4 of a resin material is applied to the surface of the pad portion 1. By the coating 4, fine particles of the resin enter minute clearances of the surface of the cloth 2, and are thereby capable of suppressing drop of waste threads and peeling of fluff of the surface, while effectively preventing threads of the cut surface from dropping. In addition, coating treatment includes coating treatment by heat seal, this coating treatment eliminates drop of waste threads and peeling of fluff of the surface in a cut portion, and it is thereby possible to eliminate a risk of development of suppuration in the cut portion after the operation. Further, the cloth 2 is not limited to nonwoven fabrics, and it is possible to use materials high in absorption.

At this point, when particles of the coating treatment with the resin material are fine particles with the diameter of 5 µmm or less, the particles enter the surface of the pad portion 1 with more excellence to form a strong coating, and provide an advantage of being hard to adhere to tissue of the body.

Examples of resin materials used in this coating treatment are silicone resins (silicone compounds), PET (polyethylene terephthalate) and PP (polypropylene), and preferable among the silicone resins and silicone compounds are silicone resins of types that the hydrophilic function is enhanced. By this means, drawing is further made ease in removing the surgical pad from the body. Accordingly, the resin materials in the surgical pad are preferably hydrophilic resins or materials containing hydrophilic resins.

Further, it is more preferable that the resin material used in the coating treatment contains an antibacterial agent. At this point, as the antibacterial agent added to the resin material, it is possible to use various substances including organic substances and inorganic substances. Organic antibacterial agents have an immediate effect, but life of antibacterial properties is short, and the agents have a defect of low heat resistance. Meanwhile, components of inorganic antibacterial agents are metal, actually used are silver, copper, zinc and zeolite, and particularly, silver is high in antibacterial effect. Therefore, among the inorganic antibacterial agents, silver antibacterial agents having silver as a main antibacterial component are preferable, life of the antibacterial effect is semipermanent, and heat resistance is excellent. At this point, since the surgical pad is disposable, life of antibacterial properties becomes almost trivial, but for heat resistance, it is necessary to consider the processing temperature in forming the resin. Accordingly, in providing the resin material covering the surface of the pad portion 1 with antibacterial properties, in view of the respects, the kind of antibacterial agent is determined.

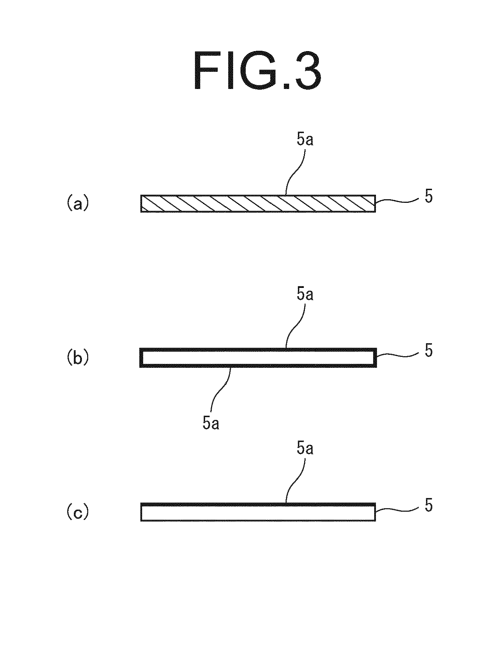

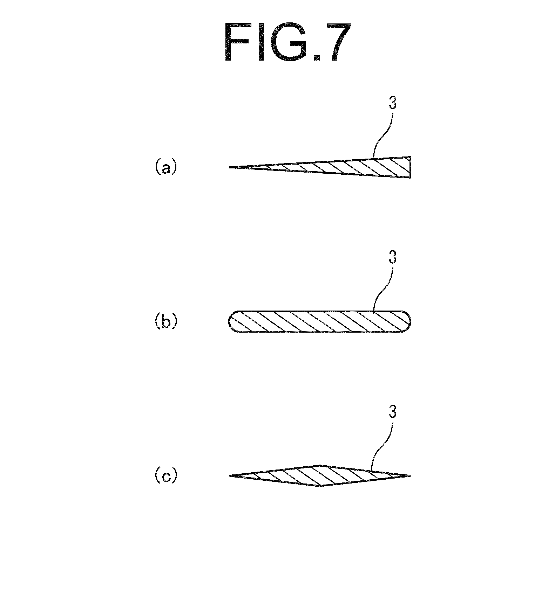

The radiopaque substance containing sheet 3 is sandwiched by layered pieces of the cloth 2, and is held along the longitudinal direction of the cloth 2. Then, as shown in FIG. 3, the radiopaque substance containing sheet 3 is formed by using a sheet-shaped resin plate as a base material 5, and containing a radiopaque substance 5a in the base material 5. To contain the radiopaque substance 5a in the base material 5, adopted is a method of kneading the radiopaque substance 5a in the resin of the base material 5 to form (FIG. 3 (a)), applying coating treatment of the radiopaque substance 5a to all surfaces or one surface of the base material 5 (FIGs. 3(b) and 3(c)), or the like. As the radiopaque substance 5a, barium sulfate is used, and besides barium sulfate, titanium oxide, silver sulfate and the like are used. In addition, the shape of cross section of the radiopaque substance containing sheet 3 does not need to be particularly uniform, and as shown in FIG. 7, various shapes such as an approximate triangle (FIG. 7 (a)), rectangle (FIG. 7(b)), and rhombus (FIG. 7(C)) are considered.

By thus forming the radiopaque substance containing body in the shape of a sheet, asperities are a few as compared with the conventional surgical pad using the thread-shaped body, and the body is not hardened by absorbing blood, body fluids and the like occurring in an operation. Then, in X-ray contrast, since the radiopaque substance is captured on the surface of the sheet, it is possible to detect the surgical pad reliably. In addition, the shape of the radiopaque substance containing sheet 3 is not limited to a rectangle, and may be a circle, trapezoid, polygon and the like.

When the base material 5 of the radiopaque substance containing sheet 3 is formed from a thermoplastic resin typified by polyethylene, polypropylene, polyvinyl chloride, polystyrene and the like, and is heat-sealed between pieces of the cloth 2 to form, the cloth 2 and the radiopaque substance containing sheet 3 is capable of being held integrally without suturing. Further, at this point, the radiopaque substance containing sheet 3 becomes a reinforcing material of the pad portion 1, and is thereby capable of suppressing deformation of the cloth 2 due to absorption of body fluid when the pad is used in an operation.

The drawing thread 6 is attached to one end in the longitudinal direction of the pad portion 1, and attachment of the drawing thread 6 to the pad portion 1 may be made by a method of passing the thread through the pad portion 1 to knot, but to provide strength to some extent, the front end of the thread 6 is made a zigzag stitch in one end in the longitudinal direction of the pad portion 1 and attached. As the material of the drawing thread 6, synthetic fibers are hardened when being soaked with blood and body fluid during the operation, but natural fibers have properties of being softened, and are preferable in terms of coming into contact with tissue in the body. Then, silk is optimal among natural fibers.

Further, using a natural fiber such as silk in the drawing thread 6 is more advantageous in sewing a zigzag stitch of the front end of the drawing thread 6 in the pad portion 1. In other words, a sewn portion 7 formed in sewing a zigzag stitch forms a relatively large area on the surface of the pad portion 1, and when the natural thread that is softened by being soaked is used, the complicated zigzag shape is prevented from injuring tissue in the body.

In the surgical pad, since the surface of the pad portion 1 is coated with the resin, even when various kinds of cloth are used in the pad portion 1 irrespective of unwoven fabric and woven fabric, it is possible to suppress drop of waste threads and peeling of fluff of the surface, and the pad is hard to adhere to tissue in the body. Then, since the radiopaque substance containing body is a film-shaped thin sheet, asperities do not occur on the surface of the pad portion 1, and further, the pad is not hardened by absorbing body fluids such as lymph, blood and cerebrospinal fluid, thereby has low friction with tissue in the body, and does not injuring the tissue.

Various dimensions are required of the pad portion 1 corresponding to the type of operation, and by preparing several kinds of base shapes of the pad portion 1 obtained by layering and integrating pieces of cloth 2 having absorption, and overlapping some pad portions 1 or folding a single pad portion 1 to overlap, it is possible to provide various types of surgical pads.

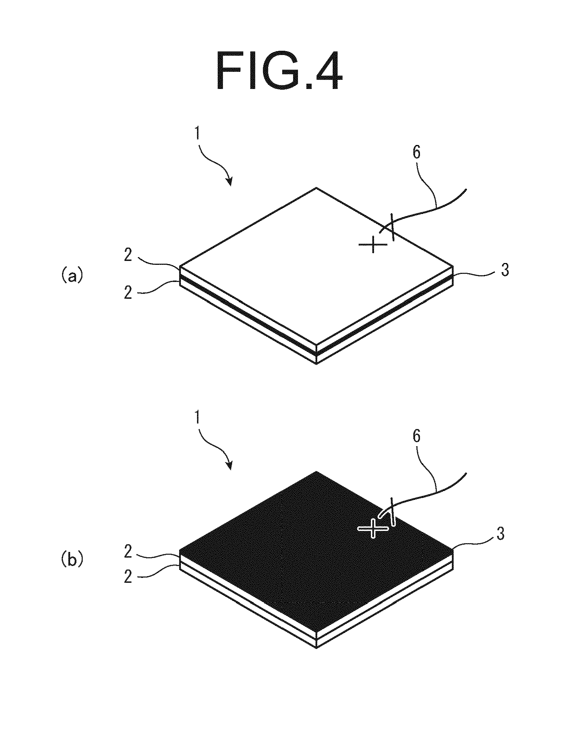

FIG. 4 shows configurations of a surgical pad according to another Embodiment. The surgical pad is small with the pad portion 1 of approximate 7 mm square and a thickness of 2 mm, and is formed by layering and integrating pieces of the cloth 2. Such a small surgical pad is used in operations of fine portions such brain surgery, and absorbs and removes a cerebrospinal fluid (spinal fluid), blood and the like. The surface of the pad portion 1 is provided with coating treatment with a resin material. The radiopaque substance containing sheet 3 has approximately the same area as the cloth 2, and the radiopaque substance is kneaded in the base material 5 of the sheet. Then, the drawing thread 6 attached to the pad portion 1 is knotted by passing the thread through the pad portion 1. The drawing thread 6 thus attached to the pad portion 1 has the function of binding the layered pieces of cloth 2 without being scattered.

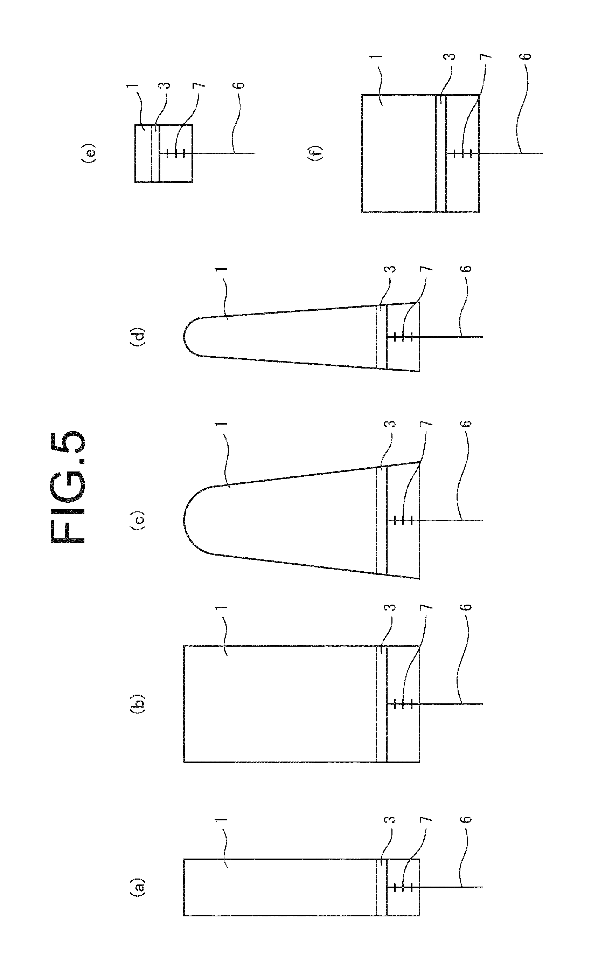

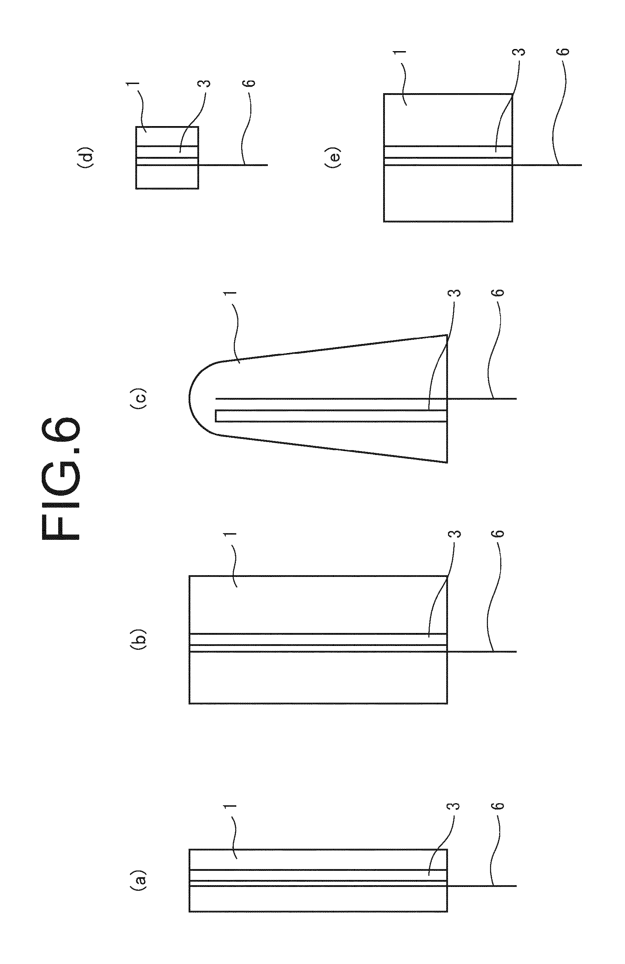

For the dimensions and shape of the above-mentioned surgical pad, it is possible to prepare various kinds corresponding to the type of operation, and further, there are various modifications of the attachment method of the drawing thread 6 to the pad portion 1 and of the manner of an arrangement of the radiopaque substance containing sheet 3. Typical examples are shown in FIGs. 5 and 6.

FIG. 5 shows examples that the drawing thread 6 and the radiopaque substance containing sheet 3 are arranged in mutually orthogonal directions, and as the shape of the pad portion 1, there are a rectangular type of a rectangle (FIGs. 5(a) and 5(b)) and square (FIGs. 5 (e) and 5(f)), and an approximate isosceles triangle type with the front end in the longitudinal direction rounded (FIGs. 5(c) and 5(d)). In any shapes, in the rectangular type having a slender shape and approximate isosceles triangle type, the drawing thread 6 is sewn in the longitudinal direction in the short end portion to form the sewn portion 7. Meanwhile, in the square type, the length of the sewn portion 7 of the drawing thread 6 is relatively long as compared with the length of the side of the square, corresponding to the surface area of the pad portion 1. This is because of ensuring the sewn portion 7 to attach the drawing thread 6 reliably.

FIG. 6 shows examples that the drawing thread 6 and the radiopaque substance containing sheet 3 are arranged in parallel with each other, and when the shape of the pad portion 1 is a rectangle (FIGs. 6 (a) and 6(b)) or an approximate isosceles triangle type with the front end in the longitudinal direction rounded (FIG. 6(c)), the radiopaque substance containing sheet 3 and the drawing thread 6 are arranged along the longitudinal direction, while being arranged along one side when the shape is a square (FIGs. 6(d) and 6(e)). It may be possible to arrange from one end to the other end of the pad portion 1 or to some midpoint. Then, in the examples of FIG. 6, the drawing thread 6 is bonded to the surface of the pad portion 1 or sandwiched between layered pieces of the cloth or the surface.

As described above, according to the present invention, the radiopaque substance containing sheet is not hardened by absorbing body fluids such as lymph, blood and cerebrospinal fluid. Then, it is possible to achieve the surgical pad capable of preventing waste threads, fluff of the surface and the like from dropping, while enabling drawing after an operation to be performed smoothly.

In addition, the present invention is not limited to the above-mentioned Embodiments, various modifications are capable of being made based on the subject matter of the invention, and the modifications are not excluded from the scope of the invention.

### Industrial Applicability

The present invention relates to a surgical pad to absorb body fluids such as blood, lymph and spinal fluid occurring from a site under an operation in surgery, and more particularly, to a surgical pad to effectively absorb a cerebrospinal fluid and the like oozing from the ventricles of the brain and subarachnoid space inside the skull without injuring brain cells in brain surgery, and has industrial applicability.

### Description of Symbols

1: Pad portion
2: Cloth
3: Radiopaque substance containing sheet
4: Coating
5: Base material
6: Drawing thread

## Claims

1. A surgical pad that absorbs body fluids such as lymph, blood and cerebrospinal fluid during surgery, comprising:
a pad portion formed by layering a plurality of pieces of cloth having absorption;
a coating portion formed from a resin on a surface of the pad portion; and
a radiopaque substance containing sheet disposed between layered pieces of cloth or on the surface of the pad portion.

2. The surgical pad according to claim 1, wherein in the radiopaque substance containing sheet, the radiopaque substance is kneaded in a base material.

3. The surgical pad according to claim 1, wherein in the radiopaque substance containing sheet, a coating of the radiopaque substance is applied to a surface of a base material.

4. The surgical pad according to claim 1, wherein the radiopaque substance containing sheet is disposed between layered pieces of cloth or on the surface of the pad portion, and is formed by containing the radiopaque substance in a sheet-shaped thermoplastic resin plate, and the thermoplastic resin plate is heat-sealed to the pad portion.

5. The surgical pad according to any one of claims 1 to 4, wherein a diameter of a particle of a resin material of the coating portion is 5 µmm or less.

6. The surgical pad according to any one of claims 1 to 4, wherein a resin material of the coating portion is a hydrophilic resin or a material containing a hydrophilic resin.

7. The surgical pad according to any one of claims 1 to 4, wherein a resin material of the coating portion contains an antibacterial agent.

8. The surgical pad according to any one of claims 1 to 7, wherein a drawing thread for a mark is attached to the pad portion.

9. The surgical pad according to claim 8, wherein the drawing thread is made of a natural material.

10. The surgical pad according to claim 9, wherein the drawing thread is made of silk.

11. The surgical pad according to any one of claims 1 to 10, wherein the cloth is comprised of a nonwoven fabric.

12. The surgical pad according to any one of claims 1 to 10, wherein the cloth is comprised of a woven fabric.
